# EUROPEAN PATENT APPLICATION

(11) **EP 3 139 154 A1**
(43) Date of publication of application: **08.03.2017**
(21) Application number: 15777282.3
(22) Date of filing: 09.04.2015
(51) Int. Cl.: G01N 21/64, G01N 21/65, G01N 33/48

(54) **SPECTROSCOPY SENSOR AND METHOD FOR MANUFACTURING SAME**

(30) Priority: 09.04.2014 KR 20140042441
(71) Applicant: Plexense, Inc., Giheung-gu, Yongin-si Gyeonggi-do 17015 (KR)
(72) Inventor: KIM, Ki Duk, Suwon-si Gyeonggi-do 443-756 (KR)
(74) Representative: V.O.
(86) International application number: PCT/KR2015/003569
(87) International publication number: WO 2015/156617

(57) **Abstract**

The present invention relates to a spectroscopic sensor and a method for manufacturing the same. A spectroscopic sensor according to one embodiment of the present invention includes a fiber layer including a plurality of flexible fibers and a surface plasmon active layer formed on the surface of the fibers. The surface plasmon active layer is densely formed and enables highly reliable spectroscopy. The fiber layer is flexible and facilitates the collection of a target sample.

## Description

### Technical Field

The present invention relates to a spectroscopic sensor, and more specifically to a spectroscopic sensor for analyzing a biological or non-biological target based on surface plasmon resonance and a method for manufacturing the spectroscopic sensor.

### Background Art

Many measurement techniques have been proposed and are commercially available for sensitive and accurate detection of target analytes in samples. Among such techniques, Raman spectroscopy and/or surface plasmon resonance (SPR) is applied to the analysis of specificity of chemical and biological molecules due to its ability to precisely measure samples at a molecular level.

The Raman spectroscopy is a measurement technique based on Raman scattering, an inelastic-scattering phenomenon, in which when incident light excites a specific light- absorbing molecule in a tangible medium while passing through the medium and is scattered, frequency shifts occur in the output light. According to the Raman spectroscopy, the vibrational energy levels of the specific light absorbing molecule are calculated from the frequency shifts in the output light caused by Raman scattering and the specific molecule is detected from the dependence of the vibrational energy of the specific light absorbing molecule on the structure and binding strength of the molecule.

Raman spectroscopy can accurately detect and define a specific molecule because it utilizes the frequency shifts inherent to the specific molecule. However, very weak signal intensities of Raman-scattered output light limit the actual application of Raman spectroscopy. In view of this limitation, surface-enhanced Raman spectroscopy (SERS) was proposed by which the weak outputs of Raman scattering are amplified to levels suitable for practical use, achieving improved detection efficiency and reproducibility. Surface-enhanced Raman spectroscopy utilizes a phenomenon in which when a specific molecule is present in the vicinity of metal nanostructures, incoming Raman laser generates surface plasmons on the surface of the metal nanostructure and the surface plasmons interact with the specific molecule to greatly amplify the Raman signals of the specific molecule. This phenomenon is referred to as "surface plasmon resonance".

A great deal of research has been conducted on the shape and/or kind of metal nanostructures for the purpose of improving the accuracy of surface-enhanced Raman spectroscopy. For SERS, metal nanoparticles are generally used as the metal nanostructures. These particles are known to amplify the Raman signals by 1 to 3 orders of magnitude when they aggregate compared to when they are isolated in the form of nanoislands. Due to their structure, such aggregates of nanoparticles can amplify the Raman signals by at least 4 orders of magnitude compared to simple metal thin films having a predetermined roughness.

The nanostructures can be produced by nanopatterning techniques, such as electron beam lithography, focused ion beam lithography, and nanoimprint lithography. However, such techniques are limited in improving the yield of the nanostructures in response to continuous processes and various substrate sizes. Mechanical contact during processing may cause defects or contamination of the nanostructures. Further, these techniques have difficulty in responding to various materials and kinds of substrates that provide surfaces on which the nanostructures are formed. For example, electron beam lithography is difficult to apply to the formation of nanostructures on substrates having a complex 3-dimensional structure because its application is limited to substrates having a 2-dimensional planar structure.

To obtain Raman scattering signals with high amplification ratio from aggregates of nanoparticles, it is necessary to uniformly form the aggregates of nanoparticles on the surface of a substrate of a SER sensor. However, uniform formation of the aggregates of nanoparticles is difficult to achieve by the techniques. Another technique was proposed in which a metal thin film having a continuous profile is deposited and is heat treated. However, this technique tends to form isolated nanoislands rather than densely aggregated nanoparticles. Another problem is that substrate materials are limited to heat resistant materials, such as glass.

### Detailed Description of the Invention

### Problems to be Solved by the Invention

Therefore, the present invention is intended to provide a spectroscopic sensor that may have a complex 3-dimensional structure, without being limited to a 2-dimensional planar structure, and includes densely formed nanostructures, enabling highly reliable spectroscopy based on surface plasmon resonance. The present invention is also intended to provide a spectroscopic sensor whose application can be extended to various targets using diverse sample collection methods.

The present invention is also intended to provide a method for manufacturing a spectroscopic sensor by which nanostructures can be densely coated on a substrate that has a complex 3-dimensional structure or is flexible in order to improve the effect on the amplification of analysis signals.

### Means for Solving the Problems

A spectroscopic sensor according to one embodiment of the present invention includes a fiber layer including a plurality of flexible fibers and a surface plasmon active layer formed on the surface of the fibers.

The fibers may form a non-woven fabric structure, a woven fabric structure, a bundle structure or a combination thereof.

The surface plasmon active layer may include conductive nanoparticles and a polymer binder immobilizing the conductive nanoparticles on the surface of the fibers. The conductive nanoparticles may form an aggregate structure or may be selected from nanospheres, nanotubes, nanocolumns, nanorods, nanopores, nanowires, and combinations thereof.

The conductive nanoparticles may include carbon particles, graphite particles, metalloid particles, metal particles, conductive metalloid oxide particles, conductive metal oxide particles, conductive metalloid nitride particles, conductive metal nitride particles, core-shell structured particles in which carbon particles, graphite particles, metalloid particles, metal particles, conductive metalloid oxide particles, conductive metal oxide particles, conductive metalloid nitride particles or conductive metal nitride particles are coated on insulating beads, or a combination thereof.

The metalloid may include antimony (Sb), germanium (Ge), arsenic (As) or an alloy thereof. The metal may be a pure metal, a transition metal or a post-transition metal and may include titanium (Ti), zinc (Zn), aluminum (Al), scandium (Sc), chromium (Cr), manganese (Mn), iron (Fe), cobalt (Co), nickel (Ni), copper (Cu), indium (In), tin (Sn), yttrium (Y), zirconium (Zr), niobium (Nb), molybdenum (Mo), ruthenium (Ru), rhodium (Rh), palladium (Pd), gold (Au), silver (Ag), platinum (Pt), strontium (Sr), tungsten (W), cadmium (Cd), tantalum (Ta) or an alloy thereof. The conductive metal oxide may include indium tin oxide (ITO), indium zinc oxide (IZO), aluminum-doped zinc oxide (AZO), gallium indium zinc oxide (GIZO), zinc oxide (ZnO) or a mixture thereof.

The surface plasmon active layer may have a thickness ranging from 10 nm to 500 nm and the fibers include either natural fibers or synthetic fibers or both of them.

The fibers may protrude from the surface of the fiber layer to provide amorphous surface plasmon fiber protrusions and the fiber layer may include pores formed between the fibers.

The polymer binder may include a cationic or anionic polymer.

The spectroscopic sensor may further include an immobilization material capable of specific binding to a target analyte on the surface plasmon active layer. The immobilization material may include at least one material selected from low molecular weight compounds, antigens, antibodies, proteins, peptides, deoxyribonucleic acid (DNA), ribonucleic acid (RNA), peptide nucleic acid (PNA), enzymes, enzyme substrates, hormones, hormone receptors, synthetic reagents having functional groups, mimics thereof, and combinations thereof.

The target analyte may be selected from amino acids, peptides, polypeptides, proteins, glycoproteins, lipoproteins, nucleosides, nucleotides, oligonucleotides, nucleic acids, sugars, carbohydrates, oligosaccharides, polysaccharides, fatty acids, lipids, hormones, metabolites, cytokines, chemokines, receptors, neurotransmitters, antigens, allergens, antibodies, substrates, metabolites, cofactors, inhibitors, drugs, pharmaceuticals, nutrients, prions, toxins, poisons, explosives, pesticides, chemical warfare agents, biological hazardous agents, bacteria, viruses, radioisotopes, vitamins, heterocyclic aromatic compounds, carcinogens, mutagens, narcotics, amphetamines, barbiturates, hallucinogens, wastes, and pollutants.

The spectroscopic sensor may be used in a surface-enhanced Raman spectroscopy (SERS), surface plasmon resonance (SPR), localized surface plasmon resonance (LSPR), absorption, and/or fluorescence mode.

A method for manufacturing a spectroscopic sensor according to a further embodiment of the present invention includes providing a mixed solution including an ionic polymer binder and conductive nanoparticles, immersing a plurality of fibers in the mixed solution, and applying an electric field to the mixed solution with the fibers immersed therein such that the conductive nanoparticles are coated on the surface of the fibers.

### Effects of the Invention

The spectroscopic sensor of the present invention includes a surface plasmon active layer formed on the surface of a fiber layer composed of a plurality of fibers. Due to the flexibility of the fibers, the spectroscopic sensor can respond to the surface shape or material of an object or organism containing an analyte. Specifically, the spectroscopic sensor can collect a sample from the object or organism by rubbing the fiber layer on the surface or penetrating the fiber layer through the surface. Therefore, the use of the spectroscopic sensor enables the measurement of various analytes and contributes to the simplification of sample preparation. In addition, the spectroscopic sensor can easily meet requirements in terms of shape, strength, transparency, porosity, heat resistance, and chemical resistance by varying the material of the fibers. Moreover, the fibers provide a larger surface area for the formation of the surface plasmon active layer thereon than any other 2-dimensional substrate and assist in the formation of the surface plasmon active layer at high density, enabling highly reliable spectroscopy based on surface plasmon resonance.

According to the method of the present invention, energy can be supplied to an ionic polymer binder and metal nanoparticles by the application of an electric field. This energy supply enables the formation of a surface plasmon active layer including highly dense nanostructures on a substrate composed of flexible fibers with a complex 3-dimensional structure in a rapid and economical manner.

### Brief Description of the Drawings

Fig. 1a is a perspective view of a spectroscopic sensor according to one embodiment of the present invention and Fig. 1b is a cross-sectional view of a plurality of fibers of the spectroscopic sensor.
Fig. 2 is a cross-sectional view illustrating a spectroscopic sensor according to one embodiment of the present invention in which a surface plasmon active layer is formed on fibers.
Fig. 3 is a cross-sectional view illustrating a spectroscopic sensor according to a further embodiment of the present invention in which a surface plasmon active layer is formed on fibers.
Fig. 4 is a flowchart illustrating a method for manufacturing a spectroscopic sensor according to one embodiment of the present invention.
Figs. 5a and 5b illustrate products obtained by the method illustrated in Fig. 4.
Fig. 6 illustrates an apparatus for manufacturing a spectroscopic sensor according to one embodiment of the present invention.
Figs. 7a and 7b are optical images of inventive and comparative spectroscopic sensors manufactured in Experimental Example 1, respectively.
Fig. 8 shows optical images of inventive spectroscopic sensors in dry and wet states.
Figs. 9a and 9b are an optical image and a scanning electron microscopy image of a spectroscopic sensor in which metal nanoparticles were coated on polycarbonate fibers, respectively.
Figs. 10a and 10b show a Raman spectrum of acetaminophen measured using an inventive spectroscopic sensor and a Raman spectrum of acetaminophen on a comparative fiber layer, respectively.

### Best Mode for Carrying out the Invention

The present invention will now be described in more detail with reference to embodiments.

Embodiments of the present invention are intended to more comprehensively explain the present invention to those skilled in the art. These embodiments may be embodied in many different forms and the scope of the invention is not limited thereto. Rather, these embodiments are provided so that this disclosure will be thorough and complete, and will fully convey the scope of the invention to those skilled in the art.

In the drawings, the thickness or size of each layer is exaggerated for convenience in description and clarity. The same reference numerals represent the same elements throughout the drawings. As used herein, the term "and/or" includes any and all combinations of one or more of the associated listed items.

The terminology used herein is for the purpose of describing particular embodiments only and is not intended to be limiting. As used herein, the singular forms "a," "an" and "the" are intended to include the plural forms as well, unless the context clearly indicates otherwise. It will be further understood that the terms "comprises" and/or "comprising," or "includes" and/or "including" when used in this specification, specify the presence of stated shapes, numbers, steps, operations, members, elements, and/or groups thereof, but do not preclude the presence or addition of one or more other shapes, numbers, operations, members, elements, and/or groups thereof.

When a layer is referred to as being "on" a fiber layer or another layer, it can be directly on the fiber layer or another layer or one or more intervening layers may be present therebetween. It will also be obvious to those skilled in the art that a structure or shape arranged adjacent to another shape can overlap the adjacent shape or be arranged below the adjacent shape.

Spatially relative terms, such as "below", "above", "upper", "lower", "horizontal" and "vertical" may be used herein to describe a relationship between one element, layer or region and another element, layer or region as illustrated in the drawings. It should be understood that these terms encompass different orientations of the device in addition to the orientation depicted in the drawings.

Hereinafter, the embodiments of the present invention will be explained with reference to cross sectional views that are schematic illustrations of idealized embodiments (and intermediate structures). In the drawings, for example, the size and shape of each constituent element can be exaggerated for convenience in description and clarity and variations from the illustrated shapes are to be expected in actual embodiments. Thus, the embodiments of the present invention should not be construed as limited to particular shapes of regions as illustrated herein. The same reference numerals denote the same parts throughout the drawings.

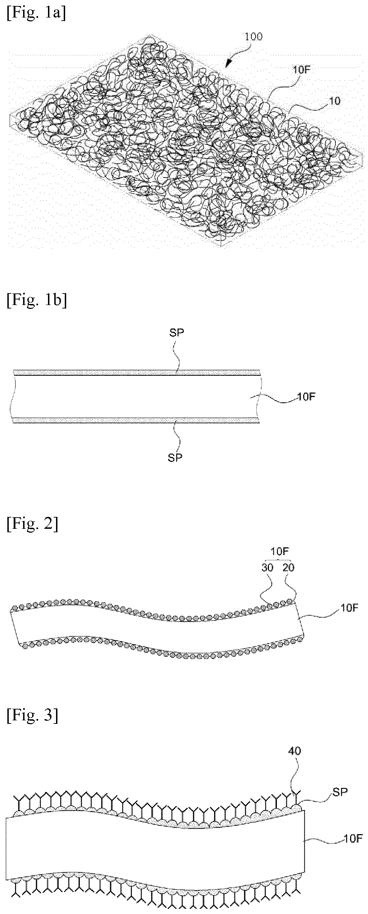

Fig. 1a is a perspective view of a spectroscopic sensor 100 according to one embodiment of the present invention and Fig. 1b is a cross-sectional view of a plurality of fibers 10F of the spectroscopic sensor.

Referring to Figs. 1a and 1b, the spectroscopic sensor 100 includes a fiber layer 10 including a plurality of fibers 10F and a surface plasmon active layer SP formed on the surface of the fibers 10F. The fibers 10F of the fiber layer 10 extend in at least two different directions, are in contact with each other, and form pores inward from the surface of the fiber layer 10. With this arrangement, the fibers 10F form a 3-dimensional structure. The pores provide passages through which a polymer binder and conductive nanoparticles can move inward from the surface of the fiber layer 10 after coated on the surface of the fibers 10F, which will be explained below.

In some embodiments, the fibers 10F may include either natural fibers or synthetic fibers or both of them. The natural fiber may be, for example, cotton, hemp, wool or silk. The synthetic fiber may be, for example, rayon, nylon, cellulose, acryl, polyester, polyethylene, polypropylene, polyethylene terephthalate, polypropylene terephthalate, polyacrylonitrile, polyethylene naphthalate, polyethersulfone, polyether ether ketone, polyphenylene sulfide, polyvinylidene fluoride or a derivative (e.g., a copolymer) thereof. However, these fiber materials are merely for illustrative purposes and the present invention is not limited thereto. For example, when a wiping mode is required for sampling, a soft, highly flexible fiber material, such as cellulose or polyester, may be used as a material for the spectroscopic sensor. Alternatively, in the case where a filtration mode is required for sampling, a strong, highly chemical resistant fiber material, such as polycarbonate, may be used.

In other embodiments, the material for the fibers 10F may be appropriately selected depending on an object and place where a sample to be collected by the spectroscopic sensor is present, the kind of the sample, and/or the type of spectroscopy employed, taking into consideration various requirements, including strength, elasticity, shrinkage, heat resistance, and chemical resistance. Other polymeric materials, petroleum pitches, and coal tar may be used for the fibers 10F.

In other embodiments, the fibers 10F may include optical fibers capable of transmitting incident light or output light for spectroscopy along the fibers. In these embodiments, the fiber layer 10 may be composed of a plurality of bundles of optical fibers or may have a woven or non-woven fabric structure in which natural fibers and/or the synthetic fibers are blended with optical fibers. For example, a plurality of optical fibers may be irregularly exposed or blended with each other between a plurality of natural fibers and/or synthetic fibers such that light is emitted outward from the surface of the fiber layer 10.

The constituent fibers 10F of the fiber layer 10 may be segmented in uniform or different lengths or may also be continuous single fibers. In some embodiments, the fibers 10F may form a non-woven fabric structure, a woven fabric structure or a combination thereof. The fiber layer 10 illustrated in Fig. 1a has a non-woven fabric texture composed of the fibers 10F. This fiber structure can also increase the density per unit volume of the fibers through a compression process.

One or more amorphous fiber protrusions may be provided on the upper surface of the fiber layer 10 to regulate the surface roughness of the fiber layer 10 or allow the fiber layer 10 to easily collect a sample.

The fiber layer 10F may be produced by randomly dispersing or mixing the fibers 10F and optionally interlacing the fibers to obtain a non-woven fabric structure. Alternatively, the fibers may be woven to obtain a regular woven fabric structure. These methods are merely representative examples and the present invention is not limited thereto. The surface plasmon active layer SP may be formed on the surface of the fibers 10F. The surface plasmon active layer SP receives light entering from the outside to generate surface plasmon resonance (SPR). The SPR amplifies signals for the detection of a target analyte in a sample, enabling reproducible and reliable spectroscopy.

For example, the surface plasmon active layer SP detects a change in plasmon resonance wavelength having a maximum absorption or scattering and/or absorbance which depends on a change in the chemical and physical environments on the surface of the surface plasmon active layer SP (for example, a change in the refractive index of a medium in contact with the surface plasmon active layer SP), so that a target analyte in a sample can be identified or the concentration of the target analyte in the sample can be determined. Surface-enhanced Raman spectroscopy (SERS) can also be performed in such a manner that surface plasmons are generated from the surface plasmon active layer SP by incident Raman laser and interact with a specific molecule of the sample to detect the specific molecule.

Due to the flexibility of the fibers 10F as substrates of the surface plasmon active layer SP, the spectroscopic sensor 100 can respond to the surface shape or material of an object or organism containing an analyte. Specifically, the spectroscopic sensor 100 can collect a sample from the object or organism in such a manner that the fiber layer 10 is rubbed on the surface of the object or organism. Thus, the spectroscopic sensor can be applied to the detection of various analytes and can contribute to the simplification of sample preparation. For example, a planar elastic substrate, such as a plastic substrate, as well as a planar inelastic substrate, such as a glass or metal substrate, should collect a sample before applied to a spectroscopic sensor due to its flexibility lower than fibers. In contrast, the spectroscopic sensor 100 can simultaneously collect and analyze a sample due to its fiber layer structure. According to an embodiment of the present invention, the spectroscopic sensor 100 can rapidly collect a liquid sample that is quickly absorbed into the fiber layer 10 through the pores of the fiber layer 10. Once absorbed through the pores, the sample can be safely maintained in the fiber layer 10 for a long time.

Fig. 2 is a cross-sectional view illustrating a spectroscopic sensor according to one embodiment of the present invention in which a surface plasmon active layer SP is formed on fibers 10F.

Referring to Fig. 2, the fibers 10F are a plurality of segmented fiber filaments or single fibers constituting a fiber layer 10. The fibers 10F and the fiber layer 10 are the same as those described with reference to Fig. 1a. The surface plasmon active layer SP formed on the surface of the fibers 10F may include conductive nanoparticles 30. The surface plasmon active layer SP may include a polymer binder 20 to immobilize the conductive nanoparticles on the surface of the fibers 10F.

The polymer binder 20 may have a thickness larger than the diameter of the conductive nanoparticles 30 on the fibers 10F. Due to these dimensions, the conductive nanoparticles can be embedded in the polymer binder 20. In some embodiments, the polymer binder 20 may have a sufficient thickness to embed aggregates of the conductive nanoparticles 30 consisting of two or more layers.

In alternative embodiments, the polymer binder 20 may have a thickness smaller than the diameter of the conductive nanoparticles 30. In these embodiments, the surface of the conductive nanoparticles 30 may be exposed to the outside of the polymer binder 20. Fig. 2 illustrates a state in which the surface of the conductive nanoparticles 30 is exposed to the outside of the polymer binder 20. The thickness of the polymer binder 20, the number of layers of the conductive nanoparticles 30, and the degree of exposure of the conductive nanoparticles 30 can be adjusted depending on the mixing ratio of the polymer binder 20 to the conductive nanoparticles in a mixed solvent and/or the concentration of the polymer binder 20 or temperature and time conditions during subsequent drying, which will be discussed later.

The molecular weight of the polymer binder 20 may be in the range of about 1,000 kDal to about 60,000 kDal. If the polymer binder 20 has a molecular weight lower than about 1,000 kDal, the adhesion of the conductive nanoparticles to the fiber layer may be insufficient. Meanwhile, if the polymer binder 20 has a molecular weight exceeding 60,000 kDal, the polymer binder 20 is excessively viscous, and as a result, the polymer binder 20 and the conductive nanoparticles 30 are not easy to deliver into the fiber layer 10 during subsequent liquid coating, making it difficult to uniformly coat the conductive nanoparticles 30 on the fibers 10F.

The polymer binder 20 may be a polymer that becomes ionic when dissolved in a suitable solvent. For example, the polymer binder 20 may be a cationic polymer and may include poly(diallydimethylammonium chloride), poly(allylamine hydrochloride), poly(4-vinylbenzyltrimethylammonium chloride), poly(ethyleneimine) or a mixture thereof. In an alternative embodiment, the polymer binder 20 may be an anionic polymer and may include poly(acrylic acid), poly(sodium 4-styrene sulfonate), poly(vinylsulfonic acid), polysodium salt, poly(amino acid) or a mixture thereof. The above-described polymers are merely representative examples and the polymer binder may be a polymer or copolymer having positive or negative ionic groups, a polymer having positive or negative ionic groups bonded to the main chain, a synthetic resin or a natural resin.

The conductive nanoparticles 30 may have an average diameter of 10 nm and 200 mm. The conductive nanoparticles 30 may be selected from nanospheres, nanotubes, nanocolumns, nanorods, nanopores, nanowires, and combinations thereof. The nanoparticles may be completely filled, porous or hollowed depending on their shape. The conductive nanoparticles may be carbon particles, graphite particles, metalloid particles, metal particles, metalloid alloy particles, metal alloy particles, conductive metal oxide particles or conductive metal nitride particles. Alternatively, the conductive nanoparticles may have a core-shell structure in which a conductive layer, such as a metal thin film, is coated on insulating glass or polymer beads. In one embodiment, the conductive nanoparticles 30 may be noble metal nanoparticles, such as gold or silver nanoparticles.

The metalloid may be antimony (Sb), germanium (Ge), arsenic (As) or an alloy thereof. The metal may be a pure metal, a transition metal or a post-transition metal. Specifically, the metal may be titanium (Ti), zinc (Zn), aluminum (Al), scandium (Sc), chromium (Cr), manganese (Mn), iron (Fe), cobalt (Co), nickel (Ni), copper (Cu), indium (In), tin (Sn), yttrium (Y), zirconium (Zr), niobium (Nb), molybdenum (Mo), ruthenium (Ru), rhodium (Rh), palladium (Pd), gold (Au), silver (Ag), platinum (Pt), strontium (Sr), tungsten (W), cadmium (Cd), tantalum (Ta) or an alloy thereof.

The conductive metal oxide may be, but not limited to, indium tin oxide (ITO), indium zinc oxide (IZO), aluminum-doped zinc oxide (AZO), gallium indium zinc oxide (GIZO) or zinc oxide (ZnO). The conductive metal nitride may be, but not limited to, tungsten nitride (WN).

In one embodiment, the surface plasmon active layer SP may have a thickness in the range of 10 nm to 500 nm. If the thickness of the surface plasmon active layer SP is less than 10 nm, the effect of the surface plasmon active layer SP on the amplification of spectroscopic signals may be negligible, and as a result, the intensities of signals for the analysis of a target analyte in a sample may be too weak to reach an analyzable level. Meanwhile, if the thickness of the surface plasmon active layer SP exceeds 500 nm, the intensities of signals for the analysis of a target analyte may be strongly amplified and the error range may increase simultaneously, resulting in low accuracy of sample analysis.

The uniform formation of the surface plasmon active layer SP on the surface of the fiber layer 10 including the fibers 10F through the internal pores ensures reproducible measurement results on a sample to be adhered to the fiber layer 10. The fibers 10F having a 1-dimensional linear structure are spatially arranged regularly or randomly to form a 3-dimensional structure. This structure allows the fiber layer to have a larger surface area than other planar substrates, such as a glass or plastic substrate, leading to an increase in the content of the surface plasmon active layer SP. As a result, the intensities of analysis signals are increased, achieving improved sensitivity.

The SPR by the conductive nanoparticles 30 enables not only surface-enhanced Raman spectroscopy (SERS) but also spectroscopy based on localized surface plasmon resonance (LSPR) caused by the nanostructures. The LSPR allows the spectroscopic sensor to detect a change in plasmon resonance wavelength having a maximum absorption or scattering rate which depends on a change in the chemical and physical environments on the surface of the nanoparticles or nanostructures (for example, a change in the refractive index of a medium in contact with the surface), so that a specific molecule can be identified or the concentration of the specific molecule in the medium can be determined. In addition, the spectroscopic sensor is highly sensitive to the refractive index change, enabling label-free detection. Therefore, the spectroscopic sensor has many advantageous over bulk SPR sensors using propagating plasmons by prism coupling.

Due to the structural characteristics of the fiber layer 10, the spectroscopic sensor of the present invention is flexible and has a large surface roughness, allowing the spectroscopic sensor to respond to the profiles of surfaces on which samples may be present. In addition, the contact surface area of the spectroscopic sensor with a sample increases. Therefore, the spectroscopic sensor can easily collect a small amount of a sample and the sensitivity to the substrate can be improved by surface plasmons.

Fig. 3 is a cross-sectional view illustrating a spectroscopic sensor according to a further embodiment in which a surface plasmon active layer SP is formed on fibers 10F. The fibers and the surface plasmon active layer SP are the same as those described above.

Referring to Fig. 3, the surface plasmon active layer SP may include conductive nanoparticles and a polymer binder immobilizing the conductive nanoparticles on the surface of the fibers 10F. As illustrated in in Fig. 3, the conductive nanoparticles aggregate in the form of a monolayer. This structure is provided for illustrative purposes only. As described in Fig. 2, the conductive nanoparticles may be provided in two or more layers. Alternatively, the conductive nanoparticles may have a discrete structure. The conductive nanoparticles may be embedded in the polymer binder or may be exposed to the outside of the polymer binder. Thus, an immobilization material 40 may be formed over the entire surface of the exposed polymer binder and/or conductive nanoparticles 30.

The immobilization material 40 can bind to a target analyte and may include at least one substance selected from low molecular weight compounds, antigens, antibodies, proteins, peptides, DNA, RNA, PNA, enzymes, enzyme substrates, hormones, hormone receptors, synthetic reagents having functional groups, mimics thereof, and combinations thereof. The binding of the immobilization material to the target analyte can be found in the art. The target analyte may be selected from amino acids, peptides, polypeptides, proteins, glycoproteins, lipoproteins, nucleosides, nucleotides, oligonucleotides, nucleic acids, sugars, carbohydrates, oligosaccharides, polysaccharides, fatty acids, lipids, hormones, metabolites, cytokines, chemokines, receptors, neurotransmitters, antigens, allergens, antibodies, substrates, metabolites, cofactors, inhibitors, drugs, pharmaceuticals, nutrients, prions, toxins, poisons, explosives, pesticides, chemical warfare agents, biological hazardous agents, bacteria, viruses, radioisotopes, vitamins, heterocyclic aromatic compounds, carcinogens, mutagens, narcotics, amphetamines, barbiturates, hallucinogens, wastes, and pollutants.

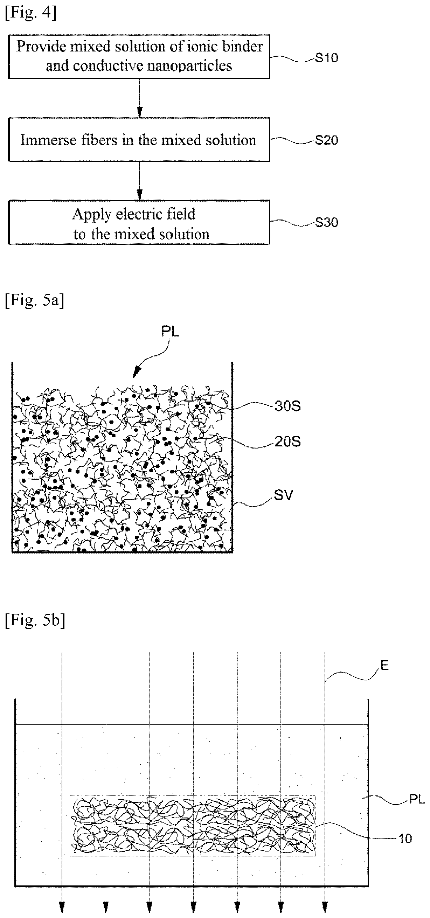

Fig. 4 is a flowchart illustrating a method for manufacturing a spectroscopic sensor according to one embodiment of the present invention and Figs. 5a and 5b illustrate products obtained by the method.

Referring to Fig. 5a in combination with Fig. 4, a mixed solution PL including an ionic polymer binder 20S and conductive nanoparticles is first prepared (S10). The mixed solution PL can be prepared by dissolving the polymer binder 20S in a suitable solvent SV and dispersing the conductive nanoparticles 30S in the solvent SV (S10). In an alternative embodiment, the polymer binder 20S may be prepared by dissolving a low molecular weight precursor, such as a monomer, in the solvent SV and polymerizing the low molecular weight precursor in the subsequent step, for example, S20 or S30, or a subsequent cross-linking step. The polymer binder 20S may get entangled with the conductive nanoparticles 30S in the mixed solution to generate a flux (or flow) of the polymer binder 20S. In this case, a flux of the conductive nanoparticles 30S may be generated in the direction of the flux of the polymer binder 20S.

The solvent SV may be selected from water, such as distilled water or deionized water, aliphatic alcohols, aliphatic ketones, aliphatic carboxylic acid esters, aliphatic carboxylic acid amides, aromatic hydrocarbons, aliphatic hydrocarbons, acetonitrile, aliphatic sulfoxides, and mixtures thereof. The solvent SV may be any known polar solvent in which the ionic polymer binder 20S can be easily dissolved.

The polymer binder 20S may be added in an amount of 0.01 to 10% by weight, based on the total weight of the mixed solution PL. The conductive nanoparticles 30S may be added in an amount of 0.01 to 10 mole%, based on the total moles of the mixed solution PL. The solvent can make up the remainder of the mixed solution. A dispersion stabilizer or a pH adjusting agent may be optionally added to the mixed solution. The dispersion stabilizer may be, for example, alginic acid, alginic acid derivatives or a mixture thereof. The pH adjusting agent may be, for example, boric acid, orthophosphoric acid, acetic acid, ascorbic acid or citric acid. The polymer binder may be photosensitive. In this case, a photoinitiator may be further added for cross-linking.

Referring to Fig. 5b in combination with Fig. 4, a fiber layer 10 is immersed in the mixed solution PL (S20). The fiber layer 10 may be arranged freely in the mixed solution PL. Alternatively, the fiber layer 10 may be fixedly positioned using a suitable tool, such as a clamp, in the mixed solution PL. The fiber layer may be provided in plurality. In an alternative embodiment, the polymer binder 20S and the conductive nanoparticles 30S may be dispersed and dissolved after immersion of the fiber layer 10 in the solvent SV.

The fiber layer 10 may be cleaned prior to the immersion step or may be surface treated. This surface treatment facilitates the attachment of a surface plasmon active layer to the fiber layer. In one embodiment, the fiber layer 10 may be coated with a photocatalyst. The photocatalyst may be prepared by a suitable process, such as a solgel process, and may be coated on the fibers 10F by spraying or application. As the photocatalyst, there may be used, for example, titanium dioxide (TiO₂), zinc oxide (ZnO) or tungsten oxide (WO₃). In an alternative embodiment, the photocatalyst may be dispersed in the mixed solution PL.

In S30, an electric field E is applied to the mixed solution PL in which the fiber layer 10 is immersed. The electric field E may have a direct current waveform, an alternating current waveform or a combination thereof. The electric field E may be a direct current electric field. In this case, the electric field E may be applied to the major surface of the fiber layer 10, for example, in a direction perpendicular to the upper surface of the fiber layer 10. The direction may be determined depending on the polarity of the ionic polymer binder 20S. For example, when it is intended to deliver the conductive nanoparticles 30S from the upper surface to the lower surface of the fiber layer 10 and the polymer binder is cationic, the electric field may be applied downward in a direction perpendicular to the fiber layer, as illustrated in Fig. 5b. In contrast, when the polymer binder is anionic, the electric field may be applied upward in a direction perpendicular to the fiber layer. In an alternative embodiment, the electric field E may be an alternating current electric field. In this embodiment, the electric field E may be applied in any direction. In another embodiment, the electric field E may be applied in different directions or a plurality of electric fields with different types of signals may be used.

The polymer binder 20S can be electrophoresed when charged by the electric field E. As a result of electrophoresis, a flux of the polymer binder 20S is generated, leading to the generation of a flux of the conductive nanoparticles 30S. The flux is activated and accelerated by the electric field E to have a higher kinetic energy so that the polymer binder 20S and the conductive nanoparticles 30S can be delivered onto the surface of the fiber layer 10 and into the fiber layer 10 through the pores of the fiber layer 10.

The kinetic energy of the conductive nanoparticles 30S and the polymer binder 20S delivered to the surface of the fibers 10F allows for the delivery of the electric field to the surface of the fibers 10F in substantially random directions. As a result, the fibers are arranged 3-dimensionally. The high kinetic energy ensures uniform coating of the conductive nanoparticles 30S and the polymer binder 20S on the surface of the fibers 10F. In addition, the conductive nanoparticles can be uniformly coated at high density on the upper surface of the fibers 10F.

In one embodiment, the fiber layer 10 may be in an electrically floating state, and the electric field E may be generated outside the mixed solution 40 and penetrate through the mixed solution 40. The electric field E may have a direct current waveform, an alternating current waveform or any other waveform, as described above, but the present invention is not limited thereto. The electric field E can be generated in a chamber by plasma discharge, which will be described below with reference to Fig. 5.

After the conductive nanoparticles 30S are sufficiently coated and immobilized on the fiber layer 10, the fiber layer 10 is recovered from the mixed solution 50. S30 requires 10 seconds to 5 minutes to complete, which is very short compared to coating with stirring. Thereafter, the fiber layer 10 may be dried. Alternatively, the fiber layer 10 may be irradiated with UV or heated to cross-link the polymer binder. In some embodiments, the fiber layer 10 may be cleaned. This cleaning removes free conductive nanoparticles or polymer binder. The polymer binder may shrink by subsequent drying. As shrinkage of the polymer binder 20 coated on the fibers 10F proceeds, the surface of the conductive nanoparticles 30 may be partially exposed, as described previously in Fig. 2.

According to the previous embodiments, a fiber layer having a non-woven fabric structure is prepared, followed by coating with metal nanoparticles. However, these embodiments are merely illustrative and the present invention is not limited thereto. For example, after coating of metal nanoparticles on a plurality of strands of fibers, the coated fibers are recovered and processed into a woven or non-woven fabric to manufacture a spectroscopic sensor in the form of a fiber layer.

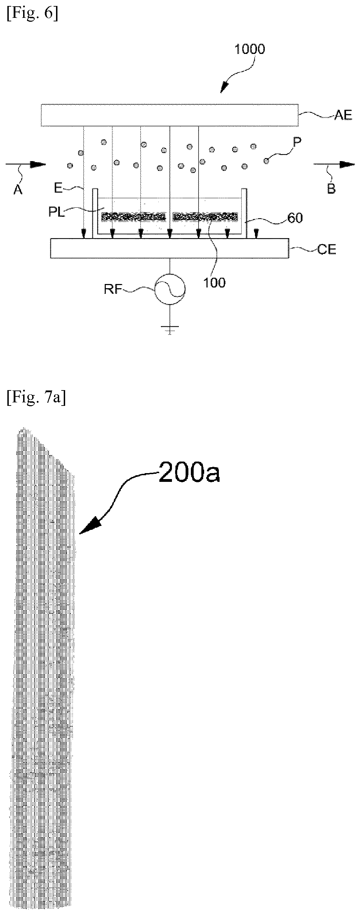

Fig. 6 illustrates an apparatus 1000 for manufacturing a spectroscopic sensor according to one embodiment of the present invention.

Referring to Fig. 6, the apparatus 1000 is an electric field (E) generator and may have two electrodes, *i.e.* an anode AE and a cathode CE, for electric field generation. Each of the anode SE and the CE may be provided in plurality. In this case, the anodes and the cathodes may also be spatially arranged such that electric fields are generated in different directions.

In one embodiment, the apparatus 1000 may optionally further include suitable gas supply means adapted to create an electric field by gas discharge. A gas P is introduced into a space defined between the anode AE and the cathode CE in the direction of an arrow A and is continuously released in the direction of an arrow B. A pump system (not shown) may be provided to decompress the space between the anode AE and the cathode CE.

The gas P may be supplied from either the anode AE or the cathode CE or both of them. For gas supply, the anode AE and the cathode CE may have through-holes similar to those of a shower head. The gas may be selected from helium (He), neon (Ne), argon (Ar), nitrogen (N₂), air, and mixtures thereof. However, these gases are merely illustrative and thus the gas P may be any other reactive gas.

An alternating current generator (RF generator) may be electrically coupled to the cathode CE to discharge the gas P, that is, to generate plasma. The anode AE may be grounded. For direct current discharge instead of alternating current discharge, a positive voltage and a negative voltage may be applied to the anode AE and the cathode CE, respectively. After a container 60 in which the fiber layer 10 is immersed in the mixed solution 50 is placed between the cathode CE and the anode AE, power is supplied to the anode AE and/or the cathode CE for several seconds to several minutes. This power supply generates plasma to immobilize the nanoparticles on the fiber layer. The container 60 and the anode AE can be maintained at a distance of 0.5 cm to 40 cm.

In the apparatus 1000 illustrated in Fig. 5, when power is applied to the alternating current generator of the cathode CE, the cathode CE has a negative potential by self-bias, and as a result, an electric field E is created between the grounded anode AE and the cathode CE in the direction of the arrows. Thus, fluxes of the conductive nanoparticles 30 and the polymer binder 20 are generated in the mixed solution. By maintaining the power supply for several seconds to several minutes, the conductive nanoparticles can be immobilized on the fiber layer 10.

As illustrated in Fig. 6, the locations of the anode AE and the cathode CE are interchangeable. The anode AE is not limited to a flat shape and may be in the form of a cover having side walls that limit a space for gas discharge or may be a chamber body. In some embodiments, the space for gas discharge may be at atmospheric pressure or a vacuum below atmospheric pressure. The vacuum may be created by a vacuum pump provided in the apparatus 1000. The plasma generator is a capacity-coupled plasma chamber but may further include any suitable inductively coupled plasma or any suitable plasma source.

### Experimental Example 1

0.01 wt% of ethylene glycol amine as a polymer binder and 0.05 wt% of gold nanoparticles (average diameter: 50 nm) as conductive nanoparticles were added to distilled water as a solvent. The mixture was stirred to prepare a mixed solution. A polyester fabric was cleaned with distilled water, followed by surface treatment. The surface-treated polyester fabric was immersed in the mixed solution.

After the mixed solution was placed in an electric field generator, an electric field was applied to the mixed solution to coat the polyester fabric with the gold nanoparticles bound to the polymer binder. To this end, a container containing the mixed solution and the polyester fabric was placed in a plasma chamber and plasma was discharged to apply an electric field to the mixed solution. After the plasma discharge was performed for 30 s, the coated fiber layer was recovered and dried to manufacture an inventive spectroscopic sensor. A comparative spectroscopic sensor was manufactured in the same manner as described above, except that no electric field was applied to the polyester fabric in the mixed solution and instead stirring was conducted for 4 h.

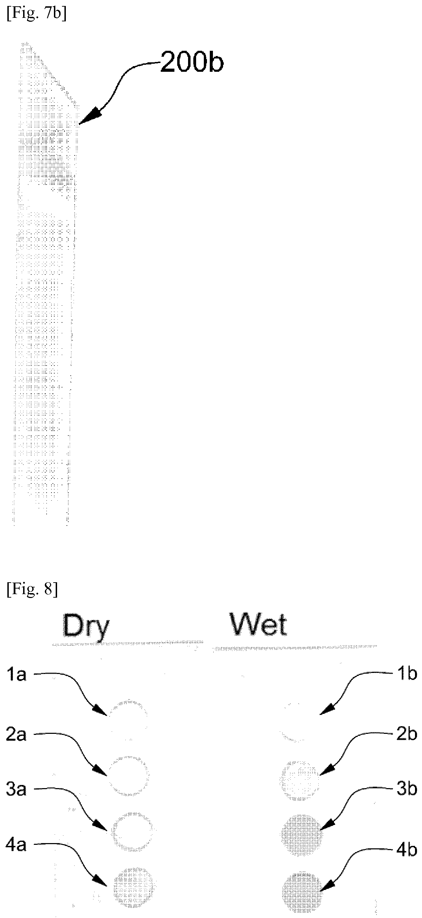

Figs. 7a and 7b are optical images of the inventive spectroscopic sensor 200a and the comparative spectroscopic sensor 200b manufactured in Experimental Example 1, respectively. Referring to Fig. 7a, the color of the inventive spectroscopic sensor 200a was darker and more uniform over its entire area than that of the comparative spectroscopic sensor 200b. These results demonstrate that the surface plasmon active layer could be uniformly and densely coated on the fiber layer in the inventive spectroscopic sensor.

Spectroscopic sensors 1a and 1b were manufactured in the same manner as in Experimental Example 1, except that the conductive nanoparticles were not coated. Spectroscopic sensors 2a and 2b were manufactured in the same manner as in Experimental Example 1. Spectroscopic sensors were manufactured in the same manner as in Experimental Example 1, except that the concentration of the conductive nanoparticles was changed by factors of 2 (3a and 3b) and 3 (4a and 4b). The coating was performed under an electric field for 30 s. Fig. 8 shows optical images of the spectroscopic sensors in dry (1a, 2a, 3a, and 4a) and wet states (1b, 2b, 3b, and 4b). These images show that the substrates for sample analysis in the dry state were darker in color than the substrates for sample analysis in the wet state.

The very high sensitivity of surface-enhanced Raman spectroscopy is explained by the amplification of surface Raman signals caused by localized surface plasmon resonance of conductive nanoparticles. In conclusion, when conductive nanoparticles are well immobilized on the surface of a substrate to exhibit localized surface plasmon resonance, the substrate can be used for a sensor that can exhibit surface-enhanced Raman resonance. Such localized surface plasmon resonance can be confirmed by measuring a change in absorbance, *i.e.* a change in the darkness of the color, after the refractive index around a substrate coated with conductive nanoparticles is artificially changed.

Fig. 8 shows changes in the color of the substrates coated with the conductive nanoparticles after exposure to air (refractive index: 1) for the sensors 1a, 2a, 3a, and 4a and exposure to water (refractive index: 1.333) for the sensors 1b, 2b, 3b, and 4b. Referring to Fig. 8, no color changes were observed in the sensor 1a whose substrate was uncoated with conductive nanoparticles and the sensor 1b whose substrate was uncoated with conductive nanoparticles and surrounded by water having a higher refractive index of 1.333. In contrast, increased absorbance values were measured for the sensors 2a, 3a, and 4a whose substrates were coated with conductive nanoparticles and surrounded by air (refractive index: 1) and remarkably increased absorbance values were measured for the sensors 2b, 3b, and 4b whose substrates were coated with conductive nanoparticles and surrounded by water having a higher refractive index of 1.333. The absorbance was found to increase with increasing amount of the conductive nanoparticles coated. Therefore, the spectroscopic sensors whose substrates were densely coated with conductive nanoparticles displayed distinct localized surface plasmon resonance and greatly amplified surface-enhanced Raman resonance, enabling accurate analysis of even small amounts of samples.

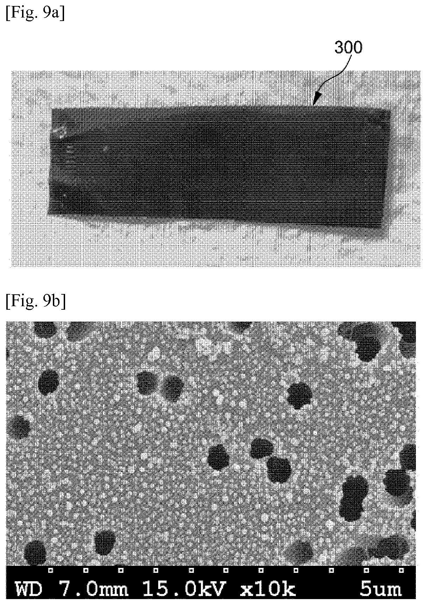

Fig. 9a and 9b are an optical image and a scanning electron microscopy image of a spectroscopic sensor 300 in which metal nanoparticles were coated on polycarbonate fibers, respectively.

In the spectroscopic sensor 300, a non-woven fabric structure based on polycarbonate fibers was coated with gold nanoparticles. Referring to Fig. 9a, the spectroscopic sensor 300 was darker in color than the spectroscopic sensors based on polycarbonate fibers coated with gold nanoparticles. The spectroscopic sensor 300 was found to have high strength and good chemical resistance. Due to these advantages, the spectroscopic sensor 300 can collect samples in both filtration and wiping modes. Fig. 9b shows that gold nanoparticles were densely coated in a binder layer of the spectroscopic sensor 300.

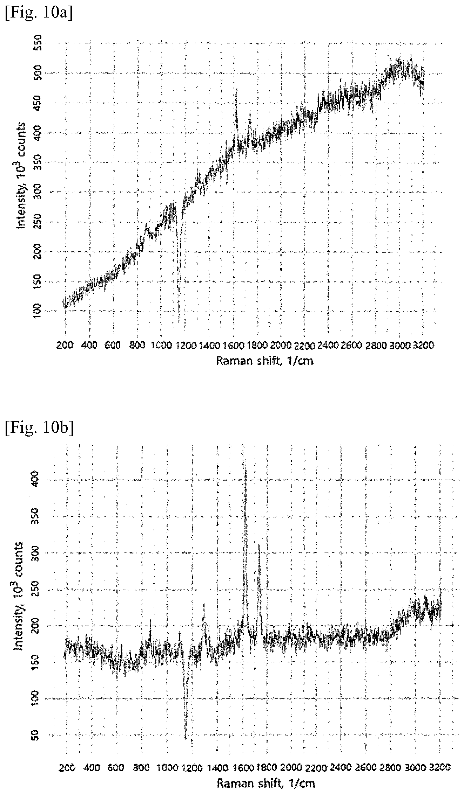

Figs. 10a and 10b show a Raman spectrum of acetaminophen measured using the inventive spectroscopic sensor and a Raman spectrum of acetaminophen on a comparative fiber layer, respectively.

Referring to Fig. 10a, Raman shift signals based on the bonding structure of acetaminophen appeared in the spectroscopic sensor based on polycarbonate fibers coated with gold nanoparticles, and their peaks were strong enough to detect. The Raman spectrum was reproducible. Referring to Fig. 10b, amplified Raman shift signals and noises were detected in the spectroscopic sensor based on polycarbonate fibers uncoated with gold nanoparticles. The Raman shift signals of Fig. 10b were more difficult to detect than those of Fig. 10a. The Raman spectrum was not reproducible.

Although the present invention has been described herein with reference to the foregoing embodiments and the accompanying drawings, the scope of the present invention is defined by the claims that follow. Accordingly, those skilled in the art will appreciate that various substitutions, modifications and changes are possible, without departing from the spirit of the present invention as disclosed in the accompanying claims.

## Claims

1. A spectroscopic sensor comprising
a fiber layer comprising a plurality of flexible fibers and
a surface plasmon active layer formed on the surface of the fibers.

2. The spectroscopic sensor according to claim 1, wherein the fibers form a non-woven fabric structure, a woven fabric structure, a bundle structure or a combination thereof.

3. The spectroscopic sensor according to claim 1, wherein the surface plasmon active layer comprises conductive nanoparticles and a polymer binder immobilizing the conductive nanoparticles on the surface of the fibers.

4. The spectroscopic sensor according to claim 3, wherein the conductive nanoparticles form an aggregate structure.

5. The spectroscopic sensor according to claim 3, wherein the conductive nanoparticles are selected from nanospheres, nanotubes, nanocolumns, nanorods, nanopores, nanowires, and combinations thereof.

6. The spectroscopic sensor according to claim 3, wherein the conductive nanoparticles comprise carbon particles, graphite particles, metalloid particles, metal particles, conductive metalloid oxide particles, conductive metal oxide particles, conductive metalloid nitride particles, conductive metal nitride particles, core-shell structured particles in which carbon particles, graphite particles, metalloid particles, metal particles, conductive metalloid oxide particles, conductive metal oxide particles, conductive metalloid nitride particles or conductive metal nitride particles are coated on insulating beads, or a combination thereof.

7. The spectroscopic sensor according to claim 6, wherein the metalloid comprises antimony (Sb), germanium (Ge), arsenic (As) or an alloy thereof; the metal is a pure metal, a transition metal or a post-transition metal and comprises titanium (Ti), zinc (Zn), aluminum (Al), scandium (Sc), chromium (Cr), manganese (Mn), iron (Fe), cobalt (Co), nickel (Ni), copper (Cu), indium (In), tin (Sn), yttrium (Y), zirconium (Zr), niobium (Nb), molybdenum (Mo), ruthenium (Ru), rhodium (Rh), palladium (Pd), gold (Au), silver (Ag), platinum (Pt), strontium (Sr), tungsten (W), cadmium (Cd), tantalum (Ta) or an alloy thereof; and the conductive metal oxide comprises indium tin oxide (ITO), indium zinc oxide (IZO), aluminum-doped zinc oxide (AZO), gallium indium zinc oxide (GIZO), zinc oxide (ZnO) or a mixture thereof.

8. The spectroscopic sensor according to claim 1, wherein the surface plasmon active layer has a thickness ranging from 10 nm to 500 nm.

9. The spectroscopic sensor according to claim 1, wherein the fibers comprise either natural fibers or synthetic fibers or both of them.

10. The spectroscopic sensor according to claim 1, wherein the fibers protrude from the surface of the fiber layer to provide amorphous surface plasmon fiber protrusions.

11. The spectroscopic sensor according to claim 1, wherein the fiber layer comprises pores formed between the fibers.

12. The spectroscopic sensor according to claim 1, wherein the polymer binder comprises a cationic or anionic polymer.

13. The spectroscopic sensor according to claim 1, further comprising an immobilization material capable of specific binding to a target analyte on the surface plasmon active layer.

14. The spectroscopic sensor according to claim 13, wherein the immobilization material comprises at least one material selected from low molecular weight compounds, antigens, antibodies, proteins, peptides, deoxyribonucleic acid (DNA), ribonucleic acid (RNA), peptide nucleic acid (PNA), enzymes, enzyme substrates, hormones, hormone receptors, synthetic reagents having functional groups, mimics thereof, and combinations thereof.

15. The spectroscopic sensor according to claim 13, wherein the target analyte is selected from amino acids, peptides, polypeptides, proteins, glycoproteins, lipoproteins, nucleosides, nucleotides, oligonucleotides, nucleic acids, sugars, carbohydrates, oligosaccharides, polysaccharides, fatty acids, lipids, hormones, metabolites, cytokines, chemokines, receptors, neurotransmitters, antigens, allergens, antibodies, substrates, metabolites, cofactors, inhibitors, drugs, pharmaceuticals, nutrients, prions, toxins, poisons, explosives, pesticides, chemical warfare agents, biological hazardous agents, bacteria, viruses, radioisotopes, vitamins, heterocyclic aromatic compounds, carcinogens, mutagens, narcotics, amphetamines, barbiturates, hallucinogens, wastes, and pollutants.

16. The spectroscopic sensor according to claim 1, wherein the spectroscopic sensor is used in a surface-enhanced Raman spectroscopy (SERS), surface plasmon resonance (SPR), localized surface plasmon resonance (LSPR), absorption, and/or fluorescence mode.

17. A method for manufacturing a spectroscopic sensor, comprising:
providing a mixed solution comprising an ionic polymer binder and conductive nanoparticles;
immersing a plurality of fibers in the mixed solution; and
applying an electric field to the mixed solution with the fibers immersed therein such that the conductive nanoparticles are coated on the surface of the fibers.

18. The method according to claim 17, wherein the fibers have a fiber layer structure before or after coating with the conductive nanoparticles and form a non-woven fabric structure, a woven fabric structure, a bundle structure or a combination thereof.

19. The method according to claim 17, wherein the fibers comprise either natural fibers or synthetic fibers or both of them.

20. The method according to claim 17, wherein the conductive nanoparticles are selected from nanospheres, nanotubes, nanocolumns, nanorods, nanopores, nanowires, and combinations thereof.

21. The method according to claim 17, wherein the conductive nanoparticles comprise carbon particles, graphite particles, metalloid particles, metal particles, conductive metalloid oxide particles, conductive metal oxide particles, conductive metalloid nitride particles, conductive metal nitride particles, core-shell structured particles in which carbon particles, graphite particles, metalloid particles, metal particles, conductive metalloid oxide particles, conductive metal oxide particles, conductive metalloid nitride particles or conductive metal nitride particles are coated on insulating beads, or a combination thereof.

22. The method according to claim 17, wherein the polymer binder comprises a cationic polymer comprising poly(diallydimethylammonium chloride), poly(allylamine hydrochloride), poly(4-vinylbenzyltrimethylammonium chloride), poly(ethyleneimine) or a mixture thereof or comprises an anionic polymer comprising poly(acrylic acid), poly(sodium 4-styrene sulfonate), poly(vinylsulfonic acid), polysodium salt, poly(amino acid) or a mixture thereof.

23. The method according to claim 17, wherein the electric field is an electrostatic field or an alternating current electric field.

24. The method according to claim 17, wherein the electric field is created by plasma discharge.

25. The method according to claim 24, wherein a gas for the plasma discharge comprises helium (He), neon (Ne), argon (Ar), nitrogen (N₂), air or a mixture thereof.
